Europäisches Patentamt

European Patent Office

Office européen des brevets

(11) Publication number: **0 439 211 A1**

# EUROPEAN PATENT APPLICATION

(21) Application number: **91200046.0**

(22) Date of filing: **12.01.91**

(51) Int. Cl.⁵: **G01N 33/569**, G01N 33/543

(30) Priority: **22.01.90 US 468050**

(43) Date of publication of application:
**31.07.91 Bulletin 91/31**

(84) Designated Contracting States:
**CH DE FR GB IT LI NL SE**

(71) Applicant: **EASTMAN KODAK COMPANY**
**343 State Street**
**Rochester, NY 14650-0221(US)**

(72) Inventor: **Snyder, Brian Anthony, c/o**
**EASTMAN KODAK COMPANY**
**Patent Department 343 State Street**
**Rochester, New York 14650-2201(US)**
Inventor: **Grogan, Elizabeth Ann, c/o**
**EASTMAN KODAK COMPANY**

**Patent Department 343 State Street**
**Rochester, New York 14650-2201(US)**
Inventor: **Zambon, Joseph James, c/o**
**EASTMAN KODAK COMPANY**
**Patent Department 343 State Street**
**Rochester, New York 14650-2201(US)**
Inventor: **Reynolds, Homer Stanley, c/o**
**EASTMAN KODAK COMPANY**
**Patent Department 343 State Street**
**Rochester, New York 14650-2201(US)**

(74) Representative: **Nunney, Ronald Frederick**
**Adolphe et al**
**Kodak Limited Patent Department Headstone**
**Drive**
**Harrow Middlesex HA1 4TY(GB)**

(54) Antibody composition for actinobacillus actinomycetemcomitans and its use in diagnostics.

(57) A polyclonal composition is useful for the immunological detection of all three serotypes (A, B and C) of Actinobacillus actinomycetemcomitans. This composition consists essentially of a polyclonal antibody directed to an antigen of serotype A, and a second polyclonal antibody directed to an antigen of serotype B. These two antibodies can be used consistently to detect all three serotypes of this microorganism. The antibody composition can be affixed to a microporous substrate for use in assays. It can also be provided as a buffered solution.

FIG. I

# ANTIBODY COMPOSITION FOR ACTINOBACILLUS ACTINOMYCETEMCOMITANS AND ITS USE IN DIAGNOSTICS

This invention relates to a polyclonal antibody composition useful for the detection of all three serotypes (A, B and C) of Actinobacillus actinomycetemcomitans. It also relates to a water-insoluble article to which the composition is affixed, and to methods of detecting the microorganism. This invention is useful in dental research and health care.

There is a continuing need in medical, dental and veterinary practices, in research and diagnostic procedures, and for rapid and accurate detection or quantification of biological or chemical substances present in biological fluids or specimens. For example, the presence of various microorganisms in human and animal tissues, fluids or cells is very important for diagnosis and effective treatment of diseases.

Specific microorganisms have been implicated as markers for a number of periodontal diseases in humans and animals, such as gingivitis and periodontitis. The importance of such diseases is growing in the human population, especially as people live longer, and prevention thereof is becoming of considerable medical and commercial importance. In addition, the proper care of animals (including dental care) is a growing concern in our culture.

Detection of microorganisms associated with periodontal diseases has been accomplished using culture techniques (which are generally slow), DNA probes and a number of immunological procedures, such as agglutination assays, enzyme linked immunosorbent assays (ELISA), immunofluorescence and immunoprecipitation. The immunological procedures involve immunological reactions of an antigenic site of the microorganism (which may be on a component extracted therefrom) with a corresponding antibody specific thereto. The resulting immunological reaction complex can then be detected in a number of ways.

Actinobacillus actinomycetemcomitans (identified herein as A. actinomycetemcomitans) is an oral gram-negative facultative organism which is closely related to the Haemophilus group of organisms. This organism can cause severe human infections, including bacterial endocarditis, thyroid gland abscess, urinary tract infection, brain abscess and vertebral osteomyelitis. It has been strongly implicated in the pathogenesis of certain types of periodontal disease, particularly in localized juvenile periodontitis and also in some forms of human periodontitis. Human isolates of A. actinomycetemcomitans have been divided into three major serogroups designated as serotypes A, B and C, corresponding to strains designated as 75 (ATCC 43717), Y4 (ATCC 43718) and 67 (ATCC 43719), respectively, by Zambon and others (Infect. Immun., 41, pp. 19-27, 1983).

Clinical assays specific to such bacteria in gingival crevicular fluid and subgingival dental plaque are useful in the diagnosis of periodontal disease, in evaluating the options for and the progress of periodontal treatment, and in determining the status of the patient at later dental examinations. The standard culture techniques used to identify such organisms are time consuming,. expensive and require a high level of operator expertise. Such tests also may lack sufficient sensitivity for detection of low levels of organisms due to strict anaerobic conditions required during transport.

The various immunoassays noted above have been developed and used with some success. Particularly useful are radioimmunoprecipitation assays and the ELISA tests. While these tests provide improvements over the standard culture techniques, further improvements have been attempted to increase assay sensitivity to the desired organism as well as to reduce cross-reactivity with other organisms which are similar, such as those in the Haemophilus group. For example, US-A-4,741,999 describes the production of monoclonal antibodies to antigens of A. actinomycetemcomitans.

While the noted monoclonal antibodies provide improved specificity for some antigens of the organism, they exhibit decreased sensitivity in detecting low levels of organisms relative to polyclonal antibodies.

There remains a need for an improved means for detecting all three serotypes of A. actinomycetemcomitans with high sensitivity and minimal cross-reactivity with antigenic sites of similar organisms.

The problems noted above are overcome with a polyclonal antibody composition useful for immunological complex formation with at least one antigen of each of the three serotypes of the microorganism Actinobacillus actinomycetemcomitans,
the composition characterised wherein it comprises, as the only immunologically reactive proteins, a first polyclonal antibody directed to serotype A of the microorganism, and a second polyclonal antibody directed to serotype B of the microorganism.

Also provided by this invention is a water-insoluble article comprising a microporous substrate having at least first and second opposing outer surfaces,
the article characterized wherein it has the polyclonal antibody composition described above affixed to at least one of the surfaces.

Moreover, a method for the detection of all three serotypes of the microorganism Actinobacillus actinomycetemcomitans comprises:

A. contacting antigen extracted from the microorganism Actinobacillus actinomycetemcomitans with the polyclonal antibody composition described above,
to form immunological complexes between the antigen and the polyclonal antibodies, and
B. detecting the presence of the complexes as an indication of the presence of the antigen.

The polyclonal composition of this invention is highly specific to all three serotypes of A. actinomycetemcomitans. Thus, only two antibodies are needed to detect all strains of this organism which may be present in a patient specimen. Normally, specific antibodies to all three serotypes would be needed to detect all three serotypes of the organism. Moreover, this composition can be used to advantage in various analytical methods including sandwich and direct binding assays (described below). While the composition can be supplied for such assays in a buffered solution, preferably it is affixed to a microporous substrate located in a water-insoluble article such as a disposable test device (described below).

FIG. 1 is a graphical plot of absolute transmittance density ($D_T$) vs. the logarithm of absolute cell concentration of A. actinomycetemcomitans exhibited in a sandwich assay. It is described in more detail in Example 3 below.

FIG. 2 is a graphical plot of absolute transmittance density ($D_T$) vs. the logarithm of absolute cell concentration of A. actinomycetemcomitans exhibited in a direct binding assay. It is described in more detail in Example 4 below.

The present invention can be used to rapidly and sensitively detect antigens (for example, lipopolysaccharides, capsule antigens or membrane proteins) of all three serotypes of A. actinomycetemcomitans simultaneously. While such antigens may be detectable as part of intact cells, normally, they are extracted in a suitable manner from the whole cells present in a biological specimen. Such specimens include, but are not limited to, saliva or mucous from the throat or mouth, urine, lacrimal fluid, human or animal tissue extracts, dental plaque and gingival crevicular fluid. Generally, the organism is detected in dental plaque, saliva, or gingival crevicular fluid.

Antigen extraction is suitably accomplished using physical or chemical means, such as by use of detergents (such as sodium dodecyl sulfate, sodium deoxycholate or sodium decyl sulfate) using standard procedures, as described, for example, in US-A-4,741,999, osmotic shock [see for example, Dirienzo and others, Infect. & Immun., 47(1), pp. 31-36, 1985], or sonic means [see for example, Zambon and others, Infect. & Immun., 41(1), pp. 19-27, 1983].

The composition of this invention consists essentially of two different polyclonal antibodies, the first directed to at least one antigen of serotype A, and the second directed to at least one antigen of serotype B, both of A. actinomycetemcomitans. These antibodies can be produced using known procedures, such as those described in the Zambon and others reference noted above. Generally, a mammal (such as a rabbit) is immunized one or more times with a suitable quantity of antigenic component or whole bacterial cells of the serotype of interest. After a suitable time, when the titer is acceptable, antisera is recovered from the mammal. Antibodies can be removed from the antisera and purified if desired using known procedures and stored in frozen buffered solutions until used. Further details of such procedures are well known in the art.

Generally, once the antibodies are isolated, they can be stored and used in buffered solutions containing one or more suitable buffers and a preservative if desired. Preferably, they are stored in frozen form. In the buffered solutions, the pH is generally maintained at from 6 to 9, and preferably at from 6.5 to 8.5. Useful buffers include phosphate buffered saline solution, tris(hydroxymethyl)aminomethane, tricine, bicine, N-tris(hydroxymethyl)methyl-2-aminoethanesulfonic acid and others readily apparent to one skilled in the art.

Preferably, the composition of polyclonal antibodies described above is immobilized in a suitable fashion on water-insoluble particulate carrier materials formed from particulate organisms, polymers, glass, ceramics, natural or synthetic resins, diatomaceous earth or magnetizable particles. These particles are preferably spherical in shape and have an average diameter of from 0.01 to 10 $\mu$m, and preferably of from 0.5 to 5 $\mu$m, although the structural and spatial configurations are not critical.

The antibodies can be attached to particulate carrier materials by physical or chemical means, including adsorption or covalent reaction with reactive groups on the surface of the materials. Covalent attachment is preferred. Preferred polymeric particles have suitable surface reactive groups for covalently attaching the antibody molecules thereto. The density of the antibody molecules on carrier materials may vary depending upon the composition of the materials, their size and the properties of the antibody, but sufficient density needed for adequate sensitivity in the assay can be readily determined by one skilled in the art. The antibodies can be modified appropriately for attachment if necessary.

Covalent attachment of antibody is usually accomplished using surface reactive groups which are capable of reacting directly or indirectly (that is through linkages, such as proteins or avidin-biotin) with free amine, carbohydrate or sulfhydryl groups of the antibody. Such surface reactive groups include, but are not limited to, carboxy, epoxy, aldehyde, active halo atoms, activated 2-substituted ethylsulfonyl, vinylsulfonyl and other groups known in the art. The following discussion regarding preferred embodiments of particles is for exemplification only, and is not meant to be limiting.

Particularly useful polymeric particles include those described in EP-A-0 323 692. Such particles are generally water-insoluble latex particles having an average particle size greater than 0.01 micrometers. They are composed of polymers prepared from one or more ethylenically unsaturated polymerizable monomers at least one of which has active halo atoms or activated 2-substituted ethylsulfonyl or vinylsulfonyl groups.

One or more of the monomers described above can be polymerized individually or in combination to form homo- or copolymers. Alternatively, and preferably, one or more of them are copolymerized with at least one other ethylenically unsaturated polymerizable monomer. Generally such monomers provide various desirable properties such as hydrophobicity, dispersibility or other features. Particularly useful comonomers are described in EP-A-0 323 692.

Representative useful polymers include the following: poly(m & p-chloromethylstyrene), poly-(styrene-co-m & p-chloromethylstyrene-co-2-hydroxyethyl acrylate) (67:30:3 molar ratio), poly-[styrene-co-m & p-(2-chloroethylsulfonylmethyl)-styrene] (96:4 molar ratio), poly(styrene- co-N-[m & p-(2-chloroethylsulfonylmethyl)phenyl]acrylamide) (99.3:0.7 molar ratio), poly(m & p-chloromethylstyrene-co-methacrylic acid)(95:5, 98:2 and 99.8:0.2 molar ratio), poly[styrene-co-m & p-(2-chloroethylsulfonylmethyl)styrene-co-methacrylic acid](93.5:4.5:2 molar ratio), poly{styrene-co-N-[m & p-(2-chloroethylsulfonylmethyl)phenyl]-acrylamide-co-methacrylic acid}(97.3:0.7:2 molar ratio), and poly(styrene-co-m & p-chloromethylstyrene)(70:30 molar ratio).

Procedures for attachment of antibodies to particles are well known in the art. Representative procedures are described, for example, in EP-A-0 323 692, US-A-3,925,157, US-A-4,181,636, US-A-4,703,018 and other references too numerous to mention. In general, the antibodies are mixed with the particles under suitable conditions depending upon the attachment form (absorption, covalent or use of linking groups). A worker skilled in the art would readily determine the appropriate conditions from the art as well as exemplary teachings presented herein. For example, for attachment to particles having reactive halo atoms, activated 2-substituted ethylsulfonyl or vinylsulfonyl groups, the antibodies are generally mixed with the particles for up to 24 hours at a temperature of from 20° to 40°C in a suspension buffered at a pH of from 7 to 10. If carboxy groups are used for reaction, the well known carbodiimide activators can be used, as well as carbamoylonium activators such as those described in EP-A-0 308 235. Antibodies can be absorbed onto particles by incubating particles and antibodies in suspension at suitable temperature for several hours.

An important aspect of the present invention is the use of a water-insoluble article to which the antibody composition of this invention is affixed. This article is composed of a microporous substrate which has first and second outer surfaces. The substrate is inert to any chemical or biological reactions and is generally composed of one or more natural or synthetic substances which have sufficient integrity for affixing the antibodies, and porosity for suitable filtration during the assay (generally an average pore size of from 0.5 to 10 μm). Useful substrate materials include, but are not limited to, natural or synthetic polymeric mats or fabrics, sintered glass, membranes or filters of glass or polymeric films or fibers, porous ceramic or cellulosic materials, porous particulate structures composed of beads bound together with an adhesive or binder material. The microporous substrate is generally flat, but some irregularities in the surface are acceptable, as well as some curvature if it is desired for the article. One skilled in the art can readily determine many commerically available materials or design others which are useful in this invention. Particularly useful materials are the polyamide (particularly, nylon) microporous membranes commercially available from Pall Corp.

The microporous substrate is designed such that it has sufficient porosity to quickly drain away fluid and uncomplexed materials encountered during an assay. Such uncomplexed materials include uncomplexed antigen, antibodies, cellular debris and other nonparticulate extraneous matter from a biological specimen. Therefore, the pore size of the microporous substrate must be such that the noted materials will pass through the porous substrate. Moreover, while some of the reagents may become entrapped within the pores of the article, the pore size generally will not accommodate a significant portion of the reagents used in the assay. It is highly preferred that substantially all of the antibody composition is on one of the outer surfaces of the microporous substrate.

If desired, the microporous substrate can be coated with proteins (such as casein or succinylated casein), as known in the art to reduce nonspecific interactions, or by surfactants to promote rapid filtration, or other optional materials which may facilitate the assay.

The antibody composition of this invention can be affixed to the microporous substrate in any suitable manner as long as the antibodies are available for reaction with antigen in the specimen. Generally, the antibodies are affixed such that moderate mechanical disturbance during manufacture and handling does not loosen them. Moreover, the antibodies generally do not come off the substrate during an assay. For example, the antibodies can be affixed mechanically by coating, spotting or spraying and held thereon by hydrophobic bonding among molecules as well as between molecules and substrate. It can also be covalently affixed by suitable chemical reaction.

It is particularly useful that the antibody composition be immobilized on the microporous substrate by attachment to particulate materials which are in turn coated on the substrate and admixed with a hydrophilic, neutral or positively-charged binder material, thereby forming a particulate composition. The amount of binder material is generally less than 20% based on the total weight of reagent.

Particularly useful binders include, but are not limited to, vinylpyrrolidone polymers, acrylamide polymers, and polymers containing quaternary salts and others known in the art which are either neutral in charge or positively charged. By "neutral in charge" is meant that either the polymer has no charges, or has a relatively small net negative or positive charge in the molecule. The polymers can be homo- or copolymers, and used singly or in combination. Acrylamide polymers are also defined to include methacrylamide polymers.

Useful positively-charged polymeric binders include mordants described in US-A-4,069,017 and references mentioned therein, provided that the mordants are sufficiently water-soluble. Sufficient water-solubility is generally provided by having at least 50% by weight of the polymer be composed of monomers containing the positively-charged groups.

Most preferably, the antibody composition of this invention is substantially on the surface of the microporous substrate, meaning that less than 5% (by weight of reagent) is entrapped within the substrate. By "entrapped" is meant that the entire composition is within a pore of the substrate. This does not mean that antibody molecules cannot be partially embedded in pores at or near the outer surface of the substrate.

The antibody composition can be affixed to a substrate surface in one or more discrete zones of the surface, each zone representing less than the total area of that surface. Alternatively, the composition can be affixed to the entire surface area.

The antibody composition can be applied to the porous substrate in any suitable manner which affixes it to the outer surface as noted above. Coating, deposition or merely dropping it onto the surface in appropriate solvents (such as water, a buffer or organic solvent which will not harm the antibodies) under suitable conditions is acceptable. Generally, the composition is dried before use in an assay although that is not necessary.

The water-insoluble article of this invention can be used in an assay without other equipment or test containers, if so desired (for example, as a hand-held article through which fluid is drained into container). Generally, however, it is mounted as part of a test device. Various test devices are known in the art including those described in US-A-3,825,410, US-A-3,888,629, US-A-3,970,429 and US-A-4,446,232. Particularly useful devices are those marketed as part of Surecell™ test kits (Eastman Kodak Company) for detection of antigenic materials. Such devices are described in EP-A-0 308 231 and EP-A-0 321 261.

More specifically, the test device comprises a water-insoluble shell having one or more test wells therein, each of which can accommodate a sample of a biological specimen and appropriate reagents.

The shell can be prepared from any useful water-insoluble material such as glass, polymeric materials, ceramics, fibrous materials, cellulosic materials and other materials known in the art.

In a preferred embodiment, the test device has three test wells designed for providing a specimen test result and positive and negative control results. Each test well has a microporous article mounted therein, and at least one of the test wells has the microporous article of the present invention mounted therein. Other variations of useful test devices would be within the purview of a worker of ordinary skill in the art.

The method of this invention can be carried out in any fashion in which the polyclonal antibody composition is contacted with a specimen suspected of containing one or more serotypes of the A. actinomycetemcomitans so that an immunological complex is formed between antibodies and any antigen present. Generally, this contact is carried out by contacting the specimen with the article of this invention which has the antibodies immobilized thereon. Contact can be accomplished in any suitable manner, but preferably, the specimen is dropped or poured onto the article, such as in a disposable test device.

Any complex formed thereby is then detected in a suitable manner as an indication of the presence of the antigen in the specimen. Detection procedures will depend upon the type of assay being carried out and the type of label used, if any with appropriate reagents and equipment.

For example, the method can be what is known as a competitive binding immunoassay in which a measured amount of labeled antigen is allowed to react with the antibodies as well as antigen in the specimen. Moreover, it is possible to indirectly measure antigen by contacting it with both labeled and unlabeled antibodies affixed to the article of this invention. The specific details of such assays are well within the purview of one of ordinary skill in the art.

In another embodiment, the antigen (preferably, extracted from the organism) in the specimen can be directly bound (or captured) on a solid support in what is called a "direct binding" assay. Once attached to the support, it can be contacted with the antibody composition of this invention to form an immunological complex bound to the support. This complex can be detected if the antibodies are labeled (such as with an enzyme), or if unlabeled, anti-antibodies directed to the first antibodies can be added to form a detectable antigen-antibody-antibody complex on the support. In either case, detection is possible using a composition which provides a dye in the presence of the enzyme and its substrate.

In a preferred embodiment, the method involves the complexation of antigen with the polyclonal antibodies of the antibody composition some of which are insolubilized, and some of which are detectably labeled but not insolubilized. The complexation with the labeled and unlabeled (insoluble) antibodies of the composition can occur in sequence, or simultaneously with binding to or collection with a separation device or solid support.

For instance, the antigen can be complexed with a first water-soluble, detectably labeled (as described above) antibody composition of this invention as well as with an insolubilized antibody composition of this invention. This is generally known in the art as an immunometric or "sandwich" assay. The labels can be any detectable moiety including specific binding reagents (such as avidin and biotin), radioisotopes or enzymes and others known in the art. More preferably the labels are enzymes (such as peroxidase, alkaline phosphatase, glucose oxidase, urease or β-galactosidase).

A preferred "sandwich" assay is described in more detail in the following example, and more generally in EP Application Serial No. _____ - (corresponding to U.S.S.Ns. 468,395, 468,046 and 468,047 filed January 22, 1990).

Alternatively, the insolubilized antibodies can be those of the polyclonal antibody composition of this invention, and the labeled antibodies can be water-soluble monoclonal or polyclonal antibodies directed to the antigen. Monoclonal antibodies and methods of production are known in the art (for example, as described in US-A-4,741,999).

In a preferred embodiment, a method for the detection of all three serotypes of the microorganism Actinobacillus actinomycetemcomitans comprises:

A. contacting a biological specimen suspected of containing extracted antigen from the microorganism Actinobacillus actinomycetemcomitans with a water-insoluble article comprising a microporous substrate having at least first and second opposing outer surfaces, and having affixed to at least one of the surfaces a polyclonal antibody composition useful for immunological complex formation with at least one antigen of each of the three serotypes of the microorganism Actinobacillus actinomycetemcomitans, the composition consisting essentially of a first polyclonal antibody directed to serotype A of the microorganism, and a second polyclonal antibody directed to serotype B of the microorganism,

to form immunological complexes between the antigen and the polyclonal antibodies, and

B. detecting the presence of the complexes as an indication of the presence of the antigen in the specimen.

Moreover, a method for the detection of all three serotypes of the microorganism Actinobacillus actinomycetemcomitans comprises:

A. contacting a biological specimen suspected of containing extracted antigen from the microorganism Actinobacillus actinomycetemcomitans with a water-insoluble article in a disposable test device, the article comprising a microporous membrane having at least first and second opposing outer surfaces, and having affixed to a discrete zone of one of the surfaces a coating of a polyclonal antibody composition consisting essentially of a first polyclonal antibody directed to serotype A of the microorganism, and a second polyclonal antibody directed to serotype B of the microorganism, both of the antibodies being immobilized on polymeric particles having an average diameter of from 0.01 to 10 meter, the particles being admixed with a hydrophilic polymeric binder material,

to form immunological complexes between the antigen and the polyclonal antibodies on the membrane,

B. prior to, simultaneously with or subsequently to contacting in step A, contacting the specimen with water-soluble enzyme-labeled antibodies to serotypes A and B to form a labeled complex of the labeled antibodies, immobilized antibodies and the antigen on the membrane,

C. simultaneously with or subsequently to contacting step B, separating uncomplexed materials from the labeled complex by washing the uncomplexed materials through the membrane, and

D. detecting the presence of the labeled complex on the membrane as an indication of the presence of the antigen in the specimen, the detection accomplished using a composition which provides a dye in the presence of the enzyme.

A. actinomycetemcomitans antigen can be extracted from a biological specimen (for example, a plaque sample) using any suitable extraction technique noted above, and preferably with 10% (by weight) of sodium dodecyl sulfate.

The extracted antigen is then contacted with the microporous article as described above to form the antigen-antibody complex with the polyclonal antibody composition of this invention affixed thereto. A brief incubation period may be desirable to enhance complex formation, but usually it is less than 5, and preferably less than 2, minutes at room temperature.

A labeled complex is formed using a detectably labeled antibody to the antigen. The labeled antibody can be reacted with the antigen prior to, simultaneously with or subsequent to contact with the antibodies on the membrane. Preferably, it is reacted subsequent to that contact.

Separation of the resulting complex from uncomplexed materials begins virtually simultaneously with complex formation. However, preferably, the reactants and fluid can be incubated on the membrane for a brief period of time to insure complete reaction. The insoluble labeled complex of antigen and two or more antibodies is retained on the membrane. One or more washes may be applied if desired to enhance separation.

The labeled complex is then suitably detected. Preferably, the label is an enzyme, and a suitable dye-forming composition is added to provide a dye (chromogen or fluorogen) in the presence of the enzyme and its substrate. Peroxidase is a preferred enzyme label, and a number of suitable dye-forming compositions are known comprising a substrate or substrate-forming reactants as well as dye-forming reactants. The substrate itself can be a dye-forming compound, such as benzidine, tetramethylbenzidine or other benzidine derivatives, 2,2'-azino-di-(3-ethyl-benzthiazolone-6-sulfonic acid), phenol red, o-phenylenediamine, pyrogallol, 4-aminoantipyrine, bromopyrogallol red and others known in the art. Alternatively, a hydrogen donor and an electron acceptor can be combined to provide a detectable species as is known in the art.

Preferably, the dye-forming composition includes a leuco dye which provides a dye in the presence of hydrogen peroxide and peroxidase (for example, a triarylimidazole leuco dye as described in US-A-4,089,747 or a triarylmethane leuco dye as described in US-A-4,670,385). A preferred dye-providing composition is illustrated in the examples below.

Once a dye has been formed in the presence of the insoluble complex, it can be evaluated visually or by using spectrophotometric equipment to determine if the assay indicates the presence of antigen in the specimen. Both positive and negative control tests may be desirably carried out with the specimen test. Appropriate reagents could be used for each control test to give the desired result.

The diagnostic test kit of this invention includes the article of this invention as well as detectably labeled antibody molecules for the antigen. These kit components can be packaged in a suitable manner and included in a carrier of some type which can be compartmentalized to receive the article (alone or in a test device) and vials or bottles of liquid or solid reagents. In addition, it can also include one or more of the following which are useful in carrying out the method: dye-forming composition, extraction reagents (if the ligand must be extracted before the assay), wash solutions, diluents, further receptor molecules and other reagents known to one skilled in the art for a given assay. Reagents can be provided in dry form or in appropriate solutions. Non-reactive components of the kit can include instructions, mixing vessels, stirring means, pipettes and the like.

In one embodiment, a diagnostic test kit useful for the determination of Actinobacillus actinomycetemcomitans comprises:

a. a water-insoluble article as described herein, and

b. a nonaffixed polyclonal antibody composition useful for immunological complex formation with at least one antigen of each of the three serotypes of the microorganism Actinobacillus actinomycetemcomitans, the composition being that described herein.

In another embodiment, a diagnostic test kit useful for the determination of Actinobacillus actinomycetemcomitans comprises:

a. a polyclonal antibody composition as described herein, and

b. detectably labeled antibodies which are directed either to the first and second polyclonal antibodies, or to all three serotypes of the microorganism Actinobacillus actinomycetemcomitans.

The following examples are presented to illustrate, and not limit, the scope of the present invention. Unless otherwise indicated, all percentages are by weight.

## Example 1: Polyclonal Antibody Composition Directed to Actinobacillus Actinomycetemcomitans

In this example, polyclonal antibodies directed to two serotypes of A. Actinomycetemcomitans were prepared. These two serotypes are designated as strains 75 (ATCC 43717) and Y4 (ATCC 43718), and referred to as serotypes A and B, respectively. ATCC represents the American Type Culture Collection (Rockville, Maryland).

The preparation of the immunogens and the immunization procedure are described by Zambon and others (Infect. Immun., 41 (1), pp. 19-27, 1983).

Antisera tc serotypes A and B were obtained from Dr. J. J. Zambon and H. S. Reynolds (SUNY, Buffalo School of Dentistry). IgG fractions of both antibodies were then prepared by ammonium sulfate precipitation and stored in admixture (at 4°C for small quantities used for research purposes, and at -70°C for larger quantities) in phosphate buffered saline solution (0.3-0.4 % solution).

## Example 2: Microporous Article Having Antibody Composition Attached Thereto

This example describes an article of the present invention having the antibody composition attached thereto for use in an assay.

The polyclonal antibody composition described in Example 1 above was immobilized onto polymeric particles comprised of poly[styrene-co-m & p-(2-chloroethylsulfonylmethyl)styrene] (96:4 molar ratio). The particles had been prepared by the procedures and using starting materials described in EP-A-0 323 692.

The antibody composition comprising antibodies to serotype A of A. actinomycetemcomitans (56.5 μg in 67 μl of phosphate buffered saline solution) and antibodies to serotype B of A. actinomycetemcomitans (56.5 μg in 45 μl of phosphate buffered saline solution) was added to a solution of borate buffer (659 μl, 0.05 molar, pH 8.5) in a test tube and mixed well. The polymeric particles (228 μl suspension containing 13.15% solids) was added to the antibody composition, and the resulting suspension was rotated end-over-end for 14-24 hours at room temperature to allow covalent attachment of antibodies to the particles. The suspension was then centrifuged at 2800 rpm for 10 minutes. The supernatant was discarded and the pellet was suspended in glycine buffer (1 ml, 0.01 molar, pH 8.5) containing merthiolate (0.01%). This procedure was repeated twice.

The final antibody/particle reagent composition contained the following:

antibody/particle reagent (1%), polyacrylamide binder (5%) and merthiolate preservative (0.010%) in glycine buffer (0.1 molar, pH 8.5). This reagent composition was then applied to a region of a LoProdyne™ or Biodyne™ A microporous membrane (Pall Corp.), and dried to form an article having the antibody composition thereon.

## Example 3: Sandwich Assay for Three Serotypes of Actinobacillus Actinomycetemcomitans

This example illustrates the use of the polyclonal composition of this invention containing antisera directed against serotypes A and B to detect all three serotypes of A. actinomycetemcomitans in a sandwich assay.

Materials:

SurecellTM disposable test devices were used in the assay. The devices each had three test wells in which there was a LoProdyne™ microporous membrane (5 μm) coated with Fluorad™ FC-135 nonionic surfactant (0.05 g/m$^2$).

Antibodies to serotypes A and B were covalently bound to polymeric particles as described in Example 2 above. The resulting antibody/particle reagent composition (2 μl) was added to each test well of the test devices.

Enzyme-antibody conjugates were prepared from both serotypes A and B antibodies, conjugated to horseradish peroxidase by the procedure of Yositake and others (Eur. J. Biochem., 101, 395, 1979). The conjugate composition comprised both conjugates (2.5 μg of each antibody/ml) in a solution comprising casein (0.5%) and merthiolate (0.01%) in 3-(N-morpholino)propanesulfonic acid buffer (0.1 molar, pH 7.5).

A dye-providing composition comprised 2-(4-hydroxy-3,5-dimethoxyphenyl)-4,5-bis(4-methoxyphenyl)imidazoleleuco dye (0.008%), poly-(vinyl pyrrolidone) (1%), sodium phosphate buffer (10 mmolar, pH 6.8) diethylenetriaminepentaacetic acid (10 μmolar), 4'-hydroxyacetanilide (2 mmolar) and hydrogen peroxide (10 mmolar).

A wash solution comprised sodium decyl sulfate in water (18 g/l).

Bacterial strains 75, Y4 and 67 were obtained from Dr. J. J. Zambon and H. S. Reynolds (identified above) corresponding to serotypes A, B and C, respectively. The antigens were extracted using sodium dodecyl sulfate (0.05%) for about one minute at room temperature.

Assay:

The extracted antigens (50 μl) from each of the serotypes A, B and C were added to separate test wells of the test devices, and allowed to complex with the antibodies immobilized on the membranes therein. Sodium dodecyl sulfate (50 µl of a 0.5%) was added to a single well of each test device as a negative control. The conjugate composition (50 µl) was added to the test wells followed by incubation at room temperature for about 5 minutes.

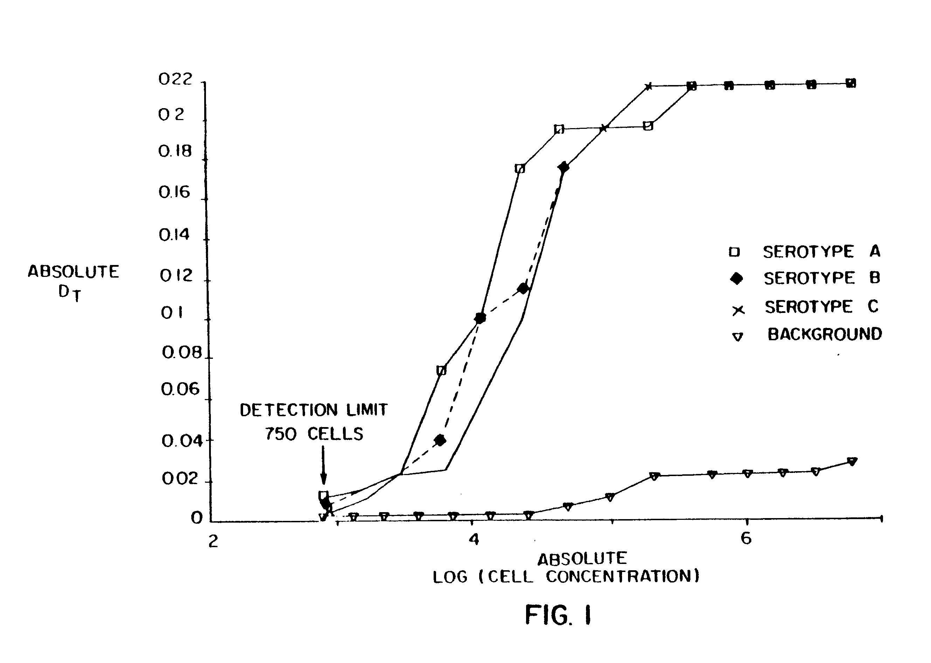

The wash solution (240 µl) was added twice to the test wells, followed by addition of the dye-providing composition (1 drop) and the test device was incubated at room temperature for two minutes. Dye formed on the membranes was visually read and compared to a calibrated color chart having reflectance density values, then converted to transmittance density (D$_T$) using the Williams-Clapper transform (J. Opt. Soc. Am., 43, 595, 1953). The results, shown in FIG. 1 as the logarithm of absolute cell concentration vs. absolute D$_T$ indicate that the composition of serotype A and B antibodies can be used to detect all strains of the microorganism in a sandwich assay. The assay can be used to detect as few as 750 cells.

Example 4: Direct Binding Assay for All Three Serotypes of Actinobacillus Actinomycetemcomitans

This example illustrates the use of the polyclonal antibody composition of this invention to detect all three serotypes of A. actinomycetemcomitans in a direct binding assay.

Materials:

SurecellTM disposable test devices were used containing 5µm BiodyneTM A microporous membranes (Pall Corp.) coated with ZonylTM FSN nonionic surfactant (0.05 g/m², DuPont).

A blocking solution comprised casein (0.5%), TweenTM 20 nonionic surfactant (0.3%), 4'-hydroxyacetanilide (0.1 molar) and merthiolate (0.01%) in 3-(N-morpholino)propanesulfonic buffer (0.01 molar, pH 7.5).

Enzyme-antibody conjugates directed against serotypes A and B, were prepared by covalently binding horseradish peroxidase to specific rabbit polyclonal antibodies by the procedure of Yositake and others (noted in Example 3). The conjugate composition comprised both conjugates (9 µg of each per ml of blocking solution).

The dye solution of Example 3 was used.

A wash solution comprised 3-cyclohexylamino-2-hydroxy-1-propanesulfonic acid buffer (0.05 molar), EmcolTM CC-9 cationic surfactant (0.75%, Witco Chemical) and merthiolate (0.01%). Its pH was adjusted to 10 by the addition of sodium hydroxide (0.05 Normal).

Bacterial strains for all three serotypes were obtained and extracted as described in Example 3 above.

Assay:

The extracted antigens (50 µl) were added to a test well of the disposable test device and allowed to adsorb to the microporous membrane. Phosphate buffered saline solution (50 µl) was added to a second well as a negative control. The enzyme-antibody conjugate solution (50 µl) was added to both test wells, followed by incubation at room temperature for 5 minutes.

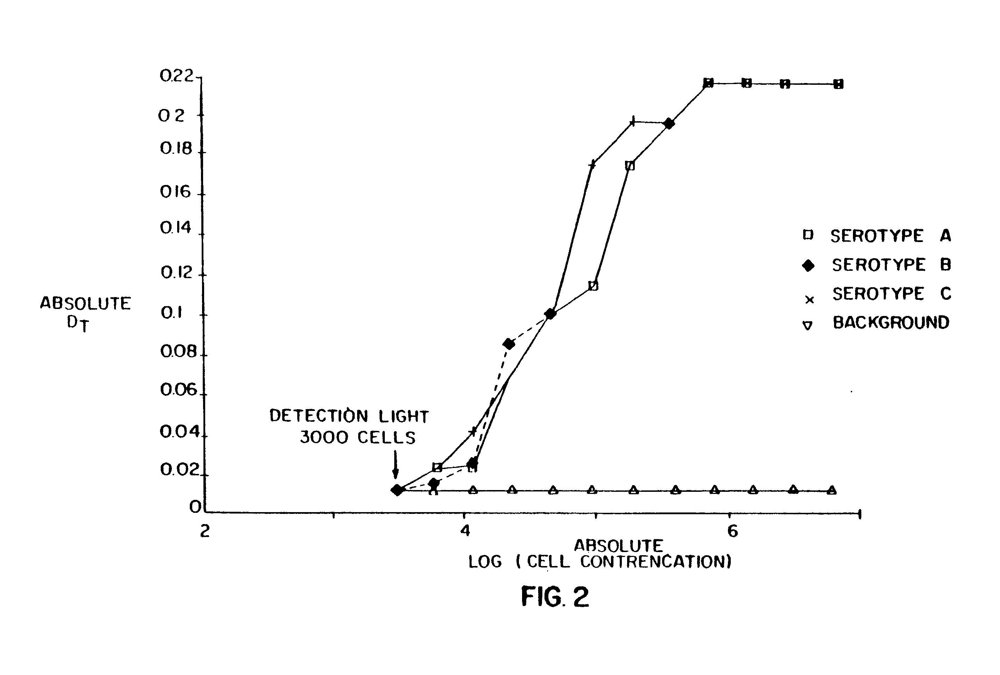

The test wells were washed twice (about 240 µl), followed by addition of the dye-providing composition (50 µl). After incubation at room temperature for two minutes, the dye on the membrane was visually observed and converted to transmittance density (D$_T$) as described in Example 3 above. The results, shown in FIG. 2 as a plot of the logarithm of cell concentration vs. absolute D$_T$, indicate that the composition of this invention can be used in a direct binding assay to detect all three serotypes of the microorganism. The assay can detect as low as about 3000 cells.

**Claims**

1. A polyclonal antibody composition useful for immunological complex formation with at least one antigen of each of the three serotypes of the microorganism Actinobacillus actinomycetemcomitans,
   the composition characterised wherein it comprises, as the only immunologically reactive proteins, a first polyclonal antibody directed to serotype A of the microorganism, and a second polyclonal antibody directed to serotype B of the microorganism.

2. The composition as claimed in claim 1 which is in a solution buffered to a pH of from 6 to 9.

3. The composition as claimed in either of claims 1 and 2 wherein each of the polyclonal antibodies is immobilized on polymeric particles.

4. A method for the detection of all three serotypes of the microorganism Actinobacillus actinomycetemcomitans comprising:
   A. contacting antigen extracted from the microorganism Actinobacillus actinomycetemcomitans with the polyclonal antibody composition as claimed in any of Claims 1 to 3, to form immunological complexes between the antigen and the polyclonal antibodies, and

B. detecting the presence of the complexes as an indication of the presence of the antigen.

5. The method as claimed in claim 4 wherein the extracted antigen is immobilized on a solid support material prior to contacting step A.

6. The method as claimed in either of claims 4 and 5 wherein the first and second polyclonal antibodies are labeled with an enzyme, and complex detection is accomplished using a composition which provides a dye in the presence of the enzyme.

7. The method as claimed in either of claims 4 and 5 wherein the polyclonal antibodies are unlabeled, and complex detection is accomplished using an enzyme-labeled antibody directed to the polyclonal antibodies.

8. A method for the detection of all three serotypes of the microorganism Actinobacillus actinomycetemcomitans comprising:

A. contacting a biological specimen suspected of containing extracted antigen from the microorganism Actinobacillus actinomycetemcomitans with a water-insoluble article comprising a microporous substrate having at least first and second opposing outer surfaces, and having affixed to at least one of the surfaces the polyclonal antibody composition as claimed in any of Claims 1 to 3,

to form immunological complexes between the antigen and the polyclonal antibodies, and

B. detecting the presence of the complexes as an indication of the presence of the antigen in the specimen.

9. A diagnostic test kit useful for the determination of Actinobacillus acetomycetemcomitans comprising:

a. the polyclonal antibody composition as claimed in any of claims 1 to 3, and

b. detectably labeled antibodies which are directed either to the first and second polyclonal antibodies, or to all three serotypes of the microorganism Actinobacillus acetomycetemcomitans.

10. A water-insoluble article comprising a microporous substrate having at least first and second opposing outer surfaces,

the article characterized wherein it has the polyclonal antibody composition as claimed in claim 1 affixed to at least one of the surfaces.

11. The article as claimed in claim 10 wherein the antibody composition is admixed with a hydrophilic neutral or positively-charged binder material.

12. The article as claimed in either of claims 10 and 11 wherein the microporous substrate is a microporous membrane having an average pore size of from 0.5 to 10 $\mu$meter.

13. A diagnostic test kit useful for the determination of Actinobacillus actinomycetemcomitans comprising:

a. the water-insoluble article as claimed in any of claims 10 to 12, and

b. a nonaffixed polyclonal antibody composition useful for immunological complex formation with at least one antigen of each of the three serotypes of the microorganism Actinobacillus actinomycetemcomitans, the composition being that claimed in either of claims 1 and 2, said first and second antibodies of said nonaffixed composition being detectably labeled.

FIG. I

EP 0 439 211 A1

FIG. 2

EP 0 439 211 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | INFECTION AND IMMUNITY, vol. 41, no. 1, July 1983, pages 19-27; J.J. ZAMBON et al.: "Serology of oral Actinobacillus actinomycetemcomitans and serotype distribution in human periodontal disease" <br> * Whole article * | 1-13 | G <br> 01 N 33/569 <br> G 01 N 33/543 |
| Y | EP-A-0 335 244   (ABBOTT LABORATORIES) <br> * Page 5, line 1 - page 6, line 40; page 8, lines 20-37 * | 1-13 | |
| A | EP-A-0 238 857   (THE RESEARCH FOUNDATION OF THE STATE UNIVERSITY OF NEW YORK) <br> * Whole document * | 1-13 | |
| | | | **TECHNICAL FIELDS SEARCHED (Int. Cl.5)** |
| | | | G 01 N |

The present search report has been drawn up for all claims

| Place of search | Date of completion of search | Examiner |
|---|---|---|
| The Hague | 22 April 91 | VAN BOHEMEN C.G. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document